(19) Europäisches Patentamt
European Patent Office
Office européen des brevets

(11) Numéro de publication: **0 323 769 B1**

(12) # FASCICULE DE BREVET EUROPEEN

(45) Date de publication de fascicule du brevet: **16.03.94**

(51) Int. Cl.[5]: **C07K 7/06**, C07K 7/08, A61K 37/02, C07K 17/00, //A61K39/385,A61K37/38

(21) Numéro de dépôt: **88402973.7**

(22) Date de dépôt: **25.11.88**

Le dossier contient des informations techniques présentées postérieurement au dépôt de la demande et ne figurant pas dans le présent fascicule.

(54) **Structures peptidiques, immunogènes les contenant et leurs applications au contrôle de la fertilité.**

(30) Priorité: **26.11.87 FR 8716420**

(43) Date de publication de la demande: **12.07.89 Bulletin 89/28**

(45) Mention de la délivrance du brevet: **16.03.94 Bulletin 94/11**

(84) Etats contractants désignés: **AT BE CH DE ES FR GB GR IT LI LU NL SE**

(56) Documents cités:
**US-A- 4 201 770**

**Decision T12/90**

**THE JOURNAL OF BIOLOGICAL CHEMISTRY, vol. 262, no. 32, 1987, pages 15483-15489, The American Society for Biochemistry and Molecular Biology, Inc.; J.M. BIDART et al.: "Immunochemical mapping of a specific domain on human choriogonadotropin using antiprotein and anti-peptide monoclonal antibodies"**

(73) Titulaire: **LAFON PHARMA S.A.**
**14 Boulevard de Perolles**
**CH-17000 Fribourg(CH)**

(72) Inventeur: **Bidart, Jean-Michel**
**172 Avenue de Choisy**
**F-75013 Paris(FR)**
Inventeur: **Bellet, Dominique**
**6 avenue Georges Pompidou**
**F-92800 Puteaux(FR)**
Inventeur: **Bohuon, Claude**
**14 Bis rue Mouton-Duvernet**
**F-75014 Paris(FR)**

(74) Mandataire: **Le Guen, Gérard et al**
**CABINET LAVOIX**
**2, place d'Estienne d'Orves**
**F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**IMMUNOLOGY TODAY, vol. 7, 1986, pages 369-374, Elsevier Science Publishers B.V., Amsterdam, NL V.C. STEVENS: "Current status of antifertility vaccines using gonadotropin immunogens"**

**BIOCHEMISTRY, vol. 24, no. 3, 1985, pages 813-816, American Society; B.B. SAXENA et al.: "Chemical synthesis of peptide fragments of the hormone-specific betasubunit of human follicle-stimulating hormone"**

**THE JOURNAL OF IMMUNULOGY, vol. 134, no. 1, January 1985, pages 457-464; Am. Ass. Imm.; US J.-M. BIDART et al. "Identification of epitopes associated with hCG and the betahCG carboxyl terminus by monoclonal antibodies produced against a synthetic peptide"**

## Description

La présente invention concerne des structures peptidiques et des immunogènes synthétiques comprenant ces structures peptidiques et leurs applications notamment comme vaccin dans un procédé de contrôle de la fertilité chez les humains et les animaux.

La présente invention concerne en particulier des vaccins anti-hCG et des vaccins anti-LH.

L'hormone chorionique gonadotrope humaine (hCG) fait partie des hormones glycoprotéiques. Quatre de ces hormones présentent une étroite parentée de structure, ce sont outre l'hCG, l'hormone lutéinisante ou lutropine (hLH), la follitropine (hFSH) et la thyrotropine (hTSH) et comprennent chacune des sous-unités $\alpha$ et $\beta$ associées par des liaisons non covalentes. La sous-unité $\alpha$ est identique dans les quatre hormones et les sous-unités $\beta$ présentent une importante homologie de structure. En particulier la $\beta$-hCG et la $\beta$-hLH ont 82% de résidus semblables et la $\beta$-hCG diffère essentiellement de la $\beta$-hLH par les 30 résidus de l'extrémité C-terminale.

Cette homologie entre les séquences existe également chez d'autres espèces et des régions hautement conservées au cours de l'évolution sont discernables non seulement pour la même hormone entre les diverses espèces (LH) mais aussi entre les hormones (CG/LH).

On rappelera en outre que les hormones CG n'existent que chez les mamifères les plus évolués et que chez les autres animaux la fonction des hormones CG est remplie par les hormones LH.

On sait que l'hCG a un rôle important dans l'établissement et le développement de la grossesse. Bien que le rôle biologique de l'hCG ne soit pas encore totalement connu il semble que son rôle principal est d'être le signal envoyé par l'ovule fécondé au corps jaune pour que celui-ci maintienne une synthèse d'hormones stéroïdiennes. Pour avoir une action biologique, l'hCG doit d'abord se fixer sur des récepteurs présents dans l'ovaire, essentiellement au niveau du corps jaune.

On a déjà cherché à inhiber la fonction de l'hCG pour disposer d'une méthode immunologique de contrôle des naissances. A cet effet, on a proposé des vaccins susceptibles d'induire la formation d'anticorps anti-hCG.

On peut citer à ce sujet le brevet FR-74 15 780 qui décrit un vaccin comprenant comme antigène, un polypeptide modifié chimiquement et notamment l'hCG ou la $\beta$-hCG modifiées chimiquement.

On peut noter également le brevet FR-75 23 673 qui décrit un vaccin, comprenant comme antigène un fragment C-terminal de la $\beta$-hCG (renfermant de 30 à 38 résidus d'acides aminés). Ce fragment a été choisi pour éviter les réactions croisées avec les hormones voisines et notamment la hLH.

On peut trouver un bilan des essais des vaccins anti-hCG dans l'article de Vernon Stevens paru dans Immunology Today (vol.7, 369, 1986). Cet article rend compte en particulier des premiers essais effectués en utilisant comme immunogène la séquence 109-145 de la $\beta$-hCG couplée à l'anatoxine diphtérique. Mais l'auteur conclut en la nécessité de développer de nouveaux vaccins anti-hCG.

Il s'avère qu'en fait l'immunogénicité de ce vaccin est faible et ne permet pas d'obtenir un taux d'anticorps suffisant.

L'Organisation Mondiale de la Santé dans un rapport de 1985 (Special programm of research development and research training in human reproduction) conclut également à la nécessité de trouver de nouveaux immunogènes.

Il est à noter par ailleurs que Aushu Vashishtha et coll. ont fait état lors du 3rd International Congress of Reproduction Immunology, Toronto 1-5 juillet 1986, de l'utilisation comme immunogène d'un peptide conjugué à l'anatoxine tétanique, ce peptide comprenant la séquence 21-31 de la $\beta$-hCG et la séquence 104-115 $\beta$-hCG-Tyr, ces deux séquences étant liées par un pont disulfure.

De plus un article de Jean Michel Bidart et coll. dans Molecular Immunology 24, 339, 1987, fait état de deux régions immuno-dominantes dans la $\beta$-hCG, à savoir la région 110-116 et la région 134-139.

On a maintenant découvert que l'on obtenait une activité immunogène importante en associant d'une part une région fortement immunogène de la $\beta$-hCG, à savoir la région autour du résidu 112 de la $\beta$-hCG et notamment la région 106-116 de la $\beta$-hCG, et d'autre part une séquence d'acides aminés comprenant un résidu lysine, tel que ceux que l'on trouve dans les séquences hautement conservées au cours de l'évolution des sous-unités $\alpha$, et qu'il était possible ainsi d'obtenir un vaccin anti-hCG.

On a également trouvé que cette découverte pouvait être étendue à d'autres hormones glycoprotéiques et notamment aux hormones luteinisantes qui ont un rôle important dans la reproduction de plusieurs espèces animales (chat, chien, etc.).

La présente invention a en conséquence pour objet une structure peptique comprenant l'association

de la séquence 106-116 de la $\beta$-hCG et

d'une séquence d'au moins cinq acides aminés comprenant au moins un résidu lysine.

La séquence 106-116 de la $\beta$-hCG est la séquence HPLTCDDPRFQ*

La séquence d'au moins 5 acides aminés comprenant au moins un résidu lysine peut être notamment une séquence d'une $\alpha$-CG contenant au moins un résidu lysine, en particulier la séquence 46-55 de l'$\alpha$-hCG, la séquence 45-49 de l'$\alpha$-hCG, la séquence 43-55 de l'$\alpha$-hCG ou la séquence 41-58 ou 59 de l'$\alpha$-hCG.

Toutefois d'autres séquences sont possibles dans la mesure où elles comprennent au moins un résidu lysine. Il peut s'agir en particulier d'une séquence inverse d'une séquence de l'$\alpha$-hCG contenant au moins un résidu lysine ou même d'une séquence comprenant, en plus d'un résidu lysine, une suite quelconque d'acides aminés.

Dans la structure peptidique selon l'invention, les deux séquences peuvent être combinées sous forme d'un enchaînement linéaire, auquel cas le résidu lysine est avantageusement séparé par au moins 4 acides aminés de la séquence 106-116 de la $\beta$-hCG. Dans cet enchaînement linéaire les séquences peuvent être combinées dans un ordre quelconque, c'est-à-dire que l'on peut avoir par exemple l'enchaînement (séquence comprenant la lysine) - (séquence 106-116 $\beta$-hCG) ou l'enchaînement (séquence 106-116 $\beta$-hCG) - (séquence comprenant la lysine).

En outre dans l'enchaînement linéaire les deux séquences peuvent être séparées par une séquence intercalaire ou "spacer" comprenant de 1 à 10 acides aminés.

Les deux séquences peuvent être également combinées en étant reliées par une liaison intermoléculaire ou même intramoléculaire. Ainsi les deux séquences peuvent être reliées par une liaison entre le groupe -COOH d'un des deux restes acide aspartique et le groupe $NH_2$ libre du résidu lysine. Dans le cas d'une liaison intramoléculaire la structure peptidique a alors la forme d'une boucle.

Pour renforcer l'immunogénicité, on couple avantageusement, de façon connue, la structure peptidique à un porteur.

L'invention a donc également pour objet un immunogène synthétique comprenant la structure peptidique selon l'invention couplée à un porteur.

Les porteurs peuvent être notamment des porteurs protéiques et notamment :

1) les anatoxines :
   . tétanique et sa fraction II préparée selon le procédé de Covey et coll., Amer. J. Reprod. Immunol. Microbiol. 8,43 (1985).
   . diphtérique, coquelucheuse.
2) les vaccins anti-diarrhéiques (contre rotavirus, choléra, Shigelles, E. Coli et salmonelles).
3) les virus de la polyomélite et de la fièvre jaune inactivés par la chaleur et le rayonnement.
4) les protéines de membrane du sporozoïte de P. Falciparum.
5) la KLH (Keyhole Limpet Hemocyanin).

Le couplage peut être réalisé à l'aide d'un agent de couplage tel que le glutaraldéhyde, un carbodiïmide ou la benzidine bis-diazotée.

Le glutaraldéhyde permet un couplage entre la protéine et le résidu lysine de la séquence contenant au moins un résidu lysine.

Les carbodiimides permettent un couplage entre la protéine et un résidu acide aspartique de la séquence 106-116 d'une $\beta$-CG ou d'une $\beta$-LH (par exemple dans la séquence 106-116 de la $\beta$-hCG il existe des résidus acide aspartique en positions 111 et 112).

La benzidine bis-diazotée nécessite pour le couplage la présence d'un résidu tyrosine sur le peptide. Dans ce but un résidu tyrosine est avantageusement ajouté en bout de chaîne.

L'immunogène synthétique peut aussi être constitué selon la technique du MAP (Multiple Antigenic Peptide) décrite par D. Posnett et al (J. Biol. Chem. 263, 17-19, 1988). Dans ce cas la synthèse du peptide est effectuée sur une matrice de polylysine préalablement construite. Ce procédé permet d'obtenir un "polymère" du peptide possédant une masse moléculaire suffisante pour être immunogène.

La présente invention a en outre pour objet un vaccin anti-fertilité qui comprend une structure peptidique selon l'invention ou de préférence un immunogène synthétique selon l'invention et un véhicule Pharmaceutiquement acceptable.

La présente invention a en particulier pour objet un vaccin anti-hCG qui comprend une structure peptidique comprenant au moins

- la séquence 106-116 de la $\beta$-hCG
- une séquence d'au moins 5 acides aminés comprenant au moins un résidu lysine

ou un immunogène synthétique comprenant une telle structure peptidique couplée à un porteur et un véhicule pharmaceutiquement acceptable.

* Pour la signification des symboles des acides aminés voir en annexe.

L'immunogène synthétique couplé à son porteur est de préférence administré après mélange avec des adjuvants d'immunisation. On pourra, par exemple, mélanger l'antigène avec l'ester N-butylique (murabutide) du muramyl dipeptide (MDP; N-acétyl-glucosamine-3yl-acétyl-L-alanyl-D-isoglutamine) dilué dans une solution saline. Le mélange peut être ensuite émulsifié au moyen d'un volume égal de squalène en présence d'arlacel (excipients). Il est aussi possible d'utiliser d'autres adjuvants comme les analogues au MDP, les fractions bactériennes telles les préparations streptococciques (OK 432), ou le Biostim (O1K2) ou des préparations de lipolysaccharides modifiés (LPS), de peptidoglycannes (N-Opaca) ou de protéoglycanes (K-Pneumoniae). Pour les excipients, les émulsions eau-dans-huile sont préférables aux émulsions huile-dans-eau.

On peut également administrer l'immunogène synthétique sous forme de particules biodégradables, telles les microcapsules ou les microsphères, les préparations de liposome et les nanoparticules. Dans ce cas, l'immunogène est inclus dans la particule. Ces procédés ont pour but d'augmenter la durée d'action du vaccin.

L'administration du vaccin se fait par voie parentérale (pour les vaccins en suspension) et éventuellement par voie orale (pour les vaccins administrés sous forme particulaire). Par exemple, l'émulsion peut être injectée par voie intramusculaire dans le muscle triceps. La quantité d'immunogène utilisée pour chaque injection est variable car elle dépend de la réponse immunitaire de chaque individu. En pratique, on utilise des doses de 50-1000 $\mu$g par injection soit 1-20 $\mu$g/kg de poids corporel. Plusieurs injections sont réalisées jusqu'à obtention d'un titre en anticorps suffisant; chaque rappel est séparé du suivant par une période de 4 à 6 semaines. On contrôle la présence d'anticorps dirigés contre l'hCG et contre la protéine porteuse éventuelle cinq jours après le second rappel puis six mois après la vaccination.

Les vaccins selon l'invention peuvent en outre contenir d'autres peptides ou d'autres immunogènes.

La présente invention a également pour objet un procédé de contrôle de la fertilité qui consiste à administrer à une femelle un vaccin selon l'invention.

La présente invention a plus particulièrement pour objet un procédé de contrôle des naissances qui consiste à administrer à une femme un vaccin comprenant une structure peptidique comprenant au moins

- la séquence 106-116 de la $\beta$-hCG
- une séquence d'au moins 5 acides aminés comprenant au moins un résidu lysine

ou un immunogène synthétique comprenant une telle structure peptidique couplée à un porteur et un véhicule pharmaceutiquement acceptable.

De plus les structures peptidiques comprenant au moins

- la séquence 106-116 de la $\beta$-hCG
- une séquence d'au moins 5 acides aminés comprenant au moins un résidu lysine

peuvent être utilisées pour bloquer les récepteurs de l'hCG et empêcher le développement de la grossesse.

La présente invention a en conséquence également pour objet une composition pour l'interruption de la grossesse comprenant à titre d'ingrédient actif une structure peptidique comprenant au moins

- la séquence 106-116 de la $\beta$-hCG
- une séquence d'au moins 5 acides aminés comprenant au moins un résidu lysine.

Les structures peptidiques selon l'invention peuvent être préparées de manière classique par synthèse peptidique en phase liquide ou solide par couplages successifs des différents résidus d'acides aminés à incorporer (de l'extrémité N-terminale vers l'extrémité C-terminale en phase liquide, ou de l'extrémité C-terminale vers l'extrémité N-terminale en phase solide) et dont les extrémités N-terminales et les chaînes latérales réactives sont préalablement bloquées par des groupements appropriés, largement connus.

On peut utiliser différentes méthodes de couplage :

1. Couplage des résidus par un carbodiimide (par ex. N-cyclohexyl-N'-morpholinoéthylcarbodiimide (CMECDI), dicyclohexylcarbodiimide (DCC), N-éthyl-N'-(3-diméthyl-aminopropyl)carbodiimide (EDC)) avec ou sans catalyse (ex. 1-hydroxybenzotriazole (HOBT)) ou autre agent couplant (ex. N-éthoxycarbonyl-2 éthoxy 1,2-dihydroquinoléine (EEDQ)).
2. Utilisation des acides aminés sous forme d'anhydrides symétriques préformés.
3. Utilisation des acides aminés sous forme d'esters activés (ex. p-nitrophénylester, HOBT ester) et couplage par l'intermédiaire de DCC.

On préfère toutefois utiliser la technique de synthèse en phase solide, dite technique de Merrifield.

Selon cette technique, on utilise une résine polymère poreuse. Sur cette résine sont greffés des groupements fonctionnels, par différentes méthodes. Le premier acide aminé est alors gréffé par sa fonction carboxylique terminale; sa fonction aminée terminale est alors protégée par un groupement protecteur labile sous certaines conditions. Les chaînes latérales sont elles aussi protégées et le resteront jusqu'à la fin de la synthèse.

Après déprotection de la fonction aminée terminale de l'acide aminé fixé sur la résine, d'une part, et activation de la fonction carboxylique de l'acide aminé à coupler, d'autre part, la formation de la liaison peptidique est réalisée. Après lavage et élimination des produits de la réaction, une nouvelle déprotection peut être entreprise pour fixer un nouvel acide aminé activé.

En dernière étape a lieu le clivage du peptide, après avoir, ou en même temps, libéré les chaînes latérales de leurs différents groupements protecteurs.

Dans le cas où le groupe protecteur de la fonction aminée est le groupe t-butyloxycarbonyle (t. Boc), il peut être éliminé par traitement de la résine avec de l'acide trifluoroacétique.

Les chaînes latérales sont alors protégées par des groupements résistants à l'action de l'acide trifluoroacétique soit : tosyle pour l'arginine et l'histidine, benzyle pour l'acide aspartique, benzyl oxycarbonyle (Z) pour la lysine.

Lorsque la synthèse de la totalité de la chaîne a été effectuée, on élimine les groupes protecteurs des différents acides aminés, notamment avec une solution d'acide fluorhydrique faiblement concentré dans un mélange paracresol/sulfure de diméthyle et on détache le peptide de la résine par exemple à l'aide d'une forte concentration en acide fluorhydrique.

Les exemples suivants illustrent la présente invention.

I - Préparation des structures peptidiques

1) Préparation du peptide (séquence 46-55 de l'$\alpha$-hCG) - (séquence 106-116 de la $\beta$-hCG) (TMLVQKNVT-SHPLTCDDPRFQ).

On a réalisé la synthèse sur un synthétiseur automatique Applied Biosystem Model 410A.

La fonction amine des acides aminés a été protégée par un groupe t-Boc et la déprotection a été effectuée par l'acide trifluoroacétique.

La déprotection finale a été réalisée avec un mélange contenant 6,5 ml de sulfure de diméthyle, 1g de paracresol et 1 ml d'HF pour 1g de résine peptide pendant 1 h à 0 ° C. Le clivage du peptide a été réalisé à 0 ° C avec 10 ml de HF et 1 g de paracresol pour 1 g de résine-peptide déprotégé.

Le peptide ainsi obtenu est ensuite purifié par chromatographie d'exclusion. Il est analysé après hydrolyse acide par chromatographie d'échanges d'une HPLC et détection à la ninhydrine. La séquence est également vérifiée.

On a rassemblé dans le tableau I suivant la structure du peptide ainsi préparé ainsi que celles d'autres peptides préparés de manière analogue.

## TABLEAU I

Peptide du type Séquence à résidu Lys - Séquence 106-116 βhCG

| | Séquence à résidu Lysine | Séquence 106-116 β-hCG |
|---|---|---|
| MIMOTOPE 1 avec séquence 46-55 α-hCG | TMLVQKNVTS | HPLTCDDPRFQ |
| MIMOTOPE 2 avec séquence α inverse | STVNKQVLMT | HPLTCDDPRFQ |
| MIMOTOPE 3 avec séquence 45-49 α-hCG | KTLMLV | HPLTCDDPRFQ |
| MIMOTOPE 4 avec séquence 43-49 α-hCG | SKKTMLV | HPLTCDDPRFQ |
| MIMOTOPE 5 avec séquence 43-55 α-hCG | SKKTMLVQKNVTS | HPLTCDDPRFQ |

2) Préparation d'un peptide à résidu tyrosine.

On préparé le peptide suivant en vue de son couplage à KLH par la benzidine sur le résidu tyrosine. Ce peptide est protégé à l'extrémité C terminale sous forme $-CONH_2$
TMLVQKNVTSHPLTCDDPRFQYG-$CONH_2$.

3) Préparation d'un peptide de type MAP.

On a préparé selon la technique décrite par Posnett et al (référence déjà citée) le peptide de type MAP ayant la formule suivante

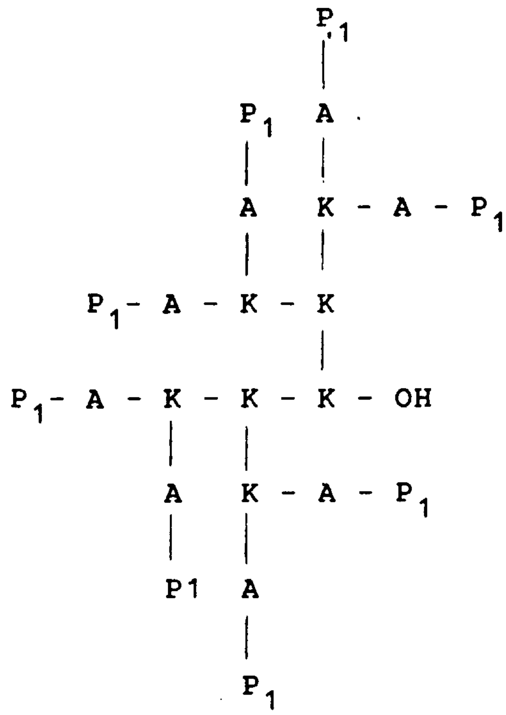

dans laquelle $P_1$ = TMLVQKNVTSHPLTCDDPRFQ (minotope 1)

II - Préparation des immunogènes synthétiques

1) Couplage avec l'anatoxine tétanique

On a couplé les structures peptidiques mimotopes avec l'anatoxine tétanique en utilisant comme agent couplant d'une part le glutaraldéhyde et d'autre part un dérivé carbodiimide.

a. Couplage au glutaraldéhyde

Cet agent couplant a été choisi pour permettre une liaison entre le groupement ξ-NH2 du résidu Lys et les groupements ξ-NH2 de la protéine porteuse.

Le schéma réactionnel est le suivant :

R1-NH2 + OCH-(CH2)3-CHO + NH2-R2 → R1-N = CH-(CH2)3-CH = N-R2

On a mis en contact le peptide et la protéine dans un rapport 50/1 dans le tampon bicarbonate 0,1 M, NaCl 0,15 M, pH = 8,5.

On a ajouté goutte à goutte le glutaraldéhyde (dilué au 1/10 dans le même tampon) de façon à avoir un rapport glutaraldéhyde/peptide de 50.

On a laissé au contact 2 heures (jaunissement de la solution sans précipitation) à température ambiante.

Puis on a effectué une chromatographie d'exclusion sur colonne Séphadex G25.

On a obtenu avec la structure peptidique minotope 1 un rapport peptide/protéine porteuse = 24.

b) Couplage avec un dérivé carbodiimide hydrosoluble

Cet agent a été choisi pour permettre une liaison majoritaire entre le β-COOH d'un résidu à groupe acide libre (acide aspartique : 111 ou 112) et les radicaux NH2 de la protéine porteuse.

On a utilisé comme carbodiimide le CMECDI.

Six milligrammes du peptide précédemment défini sont dissous dans 0,5 ml de tampon phosphate/NaCl. A cette solution, on ajoute 0,2 ml de la solution de CMECDI, ρ-méthyltoluène sulfonate (10 mg/ml). Après agitation, 5 mg de protéine porteuse (anatoxine tétanique en solution à 6 mg/ml) sont ajoutés au mélange peptide-agent couplant et incubés pendant 2h à température ambiante et sous agitation. Le conjugué peptide-protéine porteuse est ensuite purifié par une chromatographie d'exclusion et la détection du conjugué se fait grâce à un dosage des protéines par le test de Lowry. Le rapport moles de peptide/moles de protéine porteuse est déterminé au moyen d'une analyse d'acides aminés

après hydrolyse acide. Avec le mimotope 1, ce rapport est de 44 moles de peptide pour une mode de protéine porteuse.

2) Couplage avec KLH

On dissout de la benzidine(18,8 mg) dans 1,8 ml d'acide chlorhydrique 0,2 M en chauffant légèrement entre 25 ° C à 30 ° C pour obtenir une dissolution rapide et complète puis on refroidit et maintient à 0 ° C.

On ajoute goutte à goutte 200 µl d'une solution de nitrite de sodium à 14 mg/200 µl maintenue à 0 ° C.

On laisse la réaction se poursuivre environ trois minutes à 0 ° C puison utilise immédiament cette préparation de benzidine bis-diazotée.

On mélange à 0 ° C les deux solutions suivantes :

- 10 mg de KLH (Keyhole Limpet Hemocyanin; M.M:6,5x$10^6$), soit 1,5 mmole dans 750 µl de tampon borate 0,16 M pH9,1 contenant du chlorure de sodium 0,15M (la fraction insoluble de KLH est éliminée avant utilisation de la solution).
- 3 mmoles de peptide (soit 8mg pour le peptide défini sous I 2) dans 750 µl du même tampon borate pH 9,1 que précédemment.

On ajoute goutte à goutte sous agitation 100 µl de la solution de benzidine bis-diazotée.

Après seulement une minute de réaction on effectue une filtration sur gel Sephadex G.25 M (colonne Pharmacia PD10 : 50mm de hauteur sur 15mm de diamètre) équilibrée au préalable par du chlorure de sodium 0,15 M utilisé également comme phase éluante.

On recueille des fractions de 1 ml; Les fractions 3 et 4 (0,6 ml) sont conservées après avoir effectué un test à la ninhydrine sur 10µl de chaque fraction. Ces deux fractions réunies contiennent le peptide couplé sur la KLH. 100µl en sont prélevés pour une hydrolyse acide (HCl 6N + 0,1% phénol) à 110 ° C durant 18 h. Après dosage des amino-acides le ratio réel est calculé, soit ici R = 1520, ce qui correspond à 3,75 mg/ml (6 mg/1,6 ml).

III - Immunisation

On a effectué une immunisation d'au moins 2 lapins (Fauve de Bourgogne) avec chaque immunogène.

Protocole d'immunisation

1.1. Préparation de l'immunogène pour une immunisation

On a dilué dans 500 µl de tampon phosphate 0.1 M pH = 7,4 NaCl 0,15 M, 200 µg d'équivalent peptide (soit pour la structure mimotope 1 133 µl de solution protéique peptide/protéine porteuse pour le couplage au glutaraldéhyde et 167 µl pour le couplage au CME-CDI).

On a ajouté extemporanément à ces deux solutions protéiques 500 µl d'adjuvant de Freund complet ou incomplet, selon le mode d'injection.

On a émulsionné le mélange et on a procédé à l'injection.

1.2. Calendrier d'injection

| | |
|---|---|
| J0 | On a prélevé un échantillon sanguin (Témoin). Injection intradermique de la solution protéique + adjuvant de Freund complet (volume total 1 ml). 10 à 20 points d'injection (région dorsale). |
| J + 10 | Injection sous-cutanée de la solution protéique + adjuvant de Freund incomplet (volume total 1 ml). 1 seule injection. |
| J + 20 | Injection sous-cutanée de la solution protéique + adjuvant de Freund incomplet (volume total 1 ml). |
| J + 22 | Prélèvement d'un échantillon sanguin et contrôle de la présence et du titre en anticorps. |
| J + 30 | Rappel identique à J + 20. |
| J + 33 | Prélèvement identique à J + 22... |

1.3. Résultats de l'immunisation

La méthode utilisée pour détecter la présence d'anticorps dirigés contre les hormones et leurs sous-unités et leur spécificité est basée sur le dosage radioimmunologique (RIA). Les hormones (hCG, hLH) et leurs sous-unités β-hCG, α-hCG) sont marquées par un élément radioactif (NaI125) en utilisant le procédé

de Fracker P.J. et Speck, J. C. Biochem. Biophys. Res. Commun. 80,849 (1978). On incube l'antisérum à tester avec les différentes molécules marquées (traceurs) : l'antisérum est dilué dans un tampon approprié et une quantité constante de traceur est ajoutée à chaque dilution. En présence d'anticorps dans le sérum, un complexe antigène (molécule marquée)-anticorps se forme et peut être précipité par le pôlyéthylène glycol. La quantité de radioactivité précipitée est proportionnelle à la quantité d'anticorps. La spécificité des anticorps est examinée de la façon suivante : à une dilution constante de l'antisérum, on ajoute une quantité constante de hCG-I125 et des quantités croissantes de molécules à tester (hCG,β-hCG,α-hCG,hLH...). La spécificité de l'anticorps est donnée par la molécule qui, à la plus faible concentration, donne un déplacement significatif de la liaison antigène anticorps (2 résultats des immunisations chez le lapin).

Dans le tableau suivant, on donne les résultats obtenus avec les immunsérums prélevés à J22 et provenant de l'immunisation avec des immunogènes constitués par une structure peptidique selon l'invention couplée à l'anatoxine tétanique :

TABLEAU II

| | Couplage | Nombre d'animaux | Nombre de réponses positives | | | |
|---|---|---|---|---|---|---|
| | | | hCG-α | hCG-β | hCG | hLH |
| MIMOTOPE 1 avec séquence 46-55 α-hCG | α*<br>β* | 12<br>18 | 0<br>0 | 11<br>13 | 12<br>14 | 0<br>0 |
| MIMOTOPE 2 avec séquence α inverse | β | 2 | 0 | 0 | 1 | 0 |
| MIMOTOPE 4 avec séquence 43-49 α-hCG | β | 2 | 2 | 0 | 2 | 1 |
| MIMOTOPE 5 avec séquence 43-55 α-hCG | β | 2 | 1 | 2 | 2 | 1 |

* Couplage α = par le glutaraldéhyde, couplage β par carbodiimide.

Ce tableau II met en évidence les bons résultats obtenus avec le mimotope 1 avec une immunisation effective presque dans tous les cas, vis à vis de l'hCG et une absence de réactivité croisée avec la hLH.

On donnera ci-après les résultats des activités de liaison moyenne des antisérums obtenus contre le mimotope 1 à l'hCG et à ses sous-unités (moyenne et écart-type) (pourcentage après dilution au 1/10).

TABLEAU III

| Position de couplage | Nombre d'animaux | hCG-α | Liaison à : | |
|---|---|---|---|---|
| | | | hCG-β | hCG |
| α | 10 | 0 | 21(12) | 32(17)* |
| β | 10 | 0 | 21(17) | 19(15)** |

* t = 1,51
** t = 0,18
Significatif à $p<0.05$ pour $t>1,81$

Les activités de liaison mesurées vis à vis de l'hCG et sa sous-unité β montre que le couplage du peptide sur la séquence α donne une liaison des antisérums obtenus avec le mimotope 1 supérieure pour l'hCG.

IV - Activité biologique des antisérums

On a déterminé la neutralisation de l'effet biologique de l'hCG par un antisérum de lapin chez la ratte en mesurant l'inhibition de l'ovulation.

A cet effet deux rattes par groupe ont été injectées avec : 1) le sérum pré-immun, 2) le sérum immun dirigé contre le mimotope 1α obtenu chez un lapin, 3) du sérum physiologique. Huit heures après, 500 mUI

d'hCG ont été administrées. Les animaux sont sacrifiés au bout de 4 heures et on note l'apparition (+) ou l'absence (-) de follicules hémorragiques sur chaque ovaire.

| | Sérum préimmun | | Antisérum | | Sérum physiologique | |
|---|---|---|---|---|---|---|
| | ovaires | | ovaires | | ovaires | |
| | 1 | 2 | 1 | 2 | 1 | 2 |
| RATTE 1 | + | + | - | - | + | + |
| RATTE 2 | + | + | - | + | + | + |

Ces essais mettent en évidence une activité antagoniste des antisérums antimimotope 1 sur la production de follicules ovariens.

| Symboles des acides aminés | | |
|---|---|---|
| A | Ala | alanine |
| C | Cys | cystéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | serine |
| T | Thr | thréonine |
| V | Val | valine |
| W | Trp | tryptophane |
| Y | Tyr | tyrosine |

## Revendications

**Revendications pour les Etats contractants suivants : AT, BE, CH, DE, FR, GB, IT, LI, LU, NL, SE**

**1.** Structure peptidique, comprenant l'association
- de la séquence 106-116 de la $\beta$-hCG et
- d'une séquence d'au moins 5 acides aminés comprenant au moins un résidu lysine.

**2.** Structure peptidique selon la revendication 1, comprenant
- la séquence 106-116 de la $\beta$-hCG
- une séquence d'au moins 5 acides aminés d'une $\alpha$-CG comprenant au moins un résidu lysine.

**3.** Structure peptidique selon la revendication 2, comprenant
- la séquence 106-116 de la $\beta$-hCG
- la séquence 46-55 de l'$\alpha$-hCG.

**4.** Structure peptidique selon la revendication 2, comprenant
- la séquence 106-116 de la $\beta$-hCG
- la séquence 45-49 de l'$\alpha$-hCG.

5. Structure peptidique selon la revendication 2, comprenant
   - la séquence 106-116 de la β-hCG
   - la séquence 43-55 de l'α-hCG.

6. Structure peptidique selon la revendication 1 à 5, dans laquelle les deux séquences sont combinées sous forme d'un enchaînement linéaire.

7. Structure peptidique selon la revendication 6, dans laquelle le résidu lysine est séparé par au moins 4 acides aminés de la séquence 106-116 de la β-CG ou de la β-LH.

8. Structure peptidique selon la revendication 6, comprenant l'enchaînement linéaire séquence comprenant au moins un résidu lysine-séquence 106-116 de la β-hCG.

9. Structure peptidique selon la revendication 8, comprenant l'enchâinement linéaire séquence 46-55 de l'α-hCGH - séquence 106-116 de la β-hCG.

10. Immunogène synthétique comprenant une structure peptidique selon l'une quelconque des revendications précédentes, couplée à un porteur.

11. Immunogène synthétique selon la revendication 10, dans lequel le porteur est l'anatoxine tétanique.

12. Immunogène synthétique selon la revendication 10 ou la revendication 11, dans lequel le porteur est couplé avec un résidu lysine de la séquence comprenant au moins un résidu lysine.

13. Immunogène synthétique selon la revendication 12, dans lequel le couplage est réalisé par le glutaraldéhyde.

14. Immunogène synthétique selon la revendication 10 ou la revendication 11, dans lequel le porteur est couplé avec l'un des acides aminés de la séquence 106-116 de la β-hCG.

15. Immunogène synthétique selon la revendication 14, dans lequel le porteur est couplé avec l'un des résidu acide aspartique de la séquence 106-116 de la β-hCG.

16. Immunogène synthétique selon la revendication 10 comprenant une structure peptidique selon l'une quelconque des revendications 1 à 9, couplée à une matrice de polylysine.

17. Vaccin antifertilité comprenant une structure peptidique selon l'une quelconque des revendications 1 à 9 ou un immunogène synthétique selon l'une quelconque des revendications 10 à 16, et un véhicule pharmaceutiquement acceptable.

18. Vaccin anti-hCG comprenant une structure peptidique selon l'une quelconque des revendications 1 à 9 ou un immunogène synthétique comprenant une telle structure peptidique couplée à un porteur et un véhicule pharmaceutiquement acceptable.

19. Vaccin anti-hCG selon la revendication 18 pour utilisation dans un procédé de contrôle des naissances.

**Revendications pour l'Etat contractant suivant : ES**

1. Procédé de préparation d'une structure peptidique comprenant l'association
   - de la séquence 106-116 de la β-hCG et
   - d'une séquence d'au moins 5 acides aminés comprenant au moins un résidu lysine, caractérisé en ce que l'on prépare la structure par une synthèse peptidique comprenant le couplage successif des différents résidus d'acides aminés.

2. Procédé selon la revendication 1 dans lequel on prépare une structure peptidique, comprenant
   - la séquence 106-116 de la β-hCG
   - une séquence d'au moins 5 acides aminés d'une α-CG comprenant au moins un résidu Lysine.

3. Procédé selon la revendication 2 dans lequel on prépare une structure peptidique, comprenant
   - la séquence 106-116 de la β-hCG
   - la séquence 46-55 de l'α-hCG.

4. Procédé selon la revendication 2 dans lequel on prépare une structure peptidique, comprenant
   - la séquence 106-116 de la β-hCG
   - la séquence 45-49 de l'α-hCG.

5. Procédé selon la revendication 2 dans lequel on prépare une structure peptidique, comprenant
   - la séquence 106-116 de la β-hCG
   - la séquence 43-55 de l'α-hCG.

6. Procédé selon la revendication 1 dans lequel on prépare une structure peptidique dans laquelle les deux séquences sont combinées sous forme d'un enchaînement linéaire.

7. Procédé selon la revendication 6 dans lequel on prépare une structure peptidique, dans laquelle le résidu lysine est séparé par au moins 4 acides aminés de la séquence 106-116 de la β-CG ou de la β-LH.

8. Procédé selon la revendication 6 dans lequel on prépare une structure peptidique, comprenant l'enchaînement linéaire (séquence comprenant au moins un résidu lysine)-(séquence 106-116 de la β-hCG).

9. Procédé selon la revendication 8 dans lequel on prépare une structure peptidique, comprenant l'enchâînement linéaire (séquence 46-55 de l'α-hCGH) - (séquence 106-116 de la β-hCG).

10. Procédé de préparation d'un immunogène synthétique comprenant une structure peptidique obtenue par un procédé selon l'une quelconque des revendications précédentes, caractérisé en ce que l'on couple la structure peptidique à un porteur.

11. Procédé selon la revendication 10, dans lequel le porteur est l'anatoxine tétanique.

12. Procédé selon la revendication 10, dans lequel le porteur est couplé avec un résidu lysine de la séquence comprenant au moins un résidu lysine.

13. Procédé selon la revendication 12, dans lequel le couplage est réalisé par le glutaraldéhyde.

14. Procédé selon la revendication 10 ou la revendication 13, dans lequel le porteur est couplé avec l'un des acides aminés de la séquence 106-116 de la β-hCG.

15. Procédé selon la revendication 14, dans lequel le porteur est couplé avec l'un des résidus acide aspartique de la séquence 106-116 de la β-hCG.

16. Procédé de préparation d'un vaccin antifertilité, caractérisé en ce que l'on met une structure peptidique obtenue par un procédé selon l'une quelconque des revendications 1 à 9 ou un immunogène synthétique obtenu par un procédé selon l'une quelconque des revendications 10 à 15, sous une forme pharmaceutiquement acceptable.

17. Procédé de préparation d'un vaccin anti-hCG, caractérisé en ce que l'on met une structure peptidique obtenue par un procédé selon l'une quelconque des revendications 1 à 9 ou un immunogène synthétique comprenant une telle structure peptidique couplée à un porteur sous une forme pharmaceutiquement acceptable.

18. Procédé de préparation d'un immunogène synthétique caracterisé en ce que l'on synthetise une structure peptidique par un procedé selon l'une quelconque des revendications 1 à 9, sur une matrice de polylysine.

**Revendications pour l'Etat contractant suivant : GR**

**1.** Structure peptidique, comprenant l'association
de la séquence 106-116 de la β-hCG et
d'une séquence d'au moins 5 acides aminés comprenant au moins un résidu lysine.

**2.** Structure peptidique selon la revendication 1, comprenant
- la séquence 106-116 de la β-hCG
- une séquence d'au moins 5 acides aminés d'une α-CG comprenant au moins un résidu lysine.

**3.** Structure peptidique selon la revendication 2, comprenant
- la séquence 106-116 de la β-hCG
- la séquence 46-55 de l'α-hCG.

**4.** Structure peptidique selon la revendication 2, comprenant
- la séquence 106-116 de la β-hCG
- la séquence 45-49 de l'α-hCG.

**5.** Structure peptidique selon la revendication 2, comprenant
- la séquence 106-116 de la β-hCG
- la séquence 43-55 de l'α-hCG.

**6.** Structure peptidique selon la revendication 1 à 5, dans laquelle les deux séquences sont combinées sous forme d'un enchaînement linéaire.

**7.** Structure peptidique selon la revendication 6, dans laquelle le résidu lysine est séparé par au moins 4 acides aminés de la séquence 106-116 de la β-CG ou de la β-LH.

**8.** Structure peptidique selon la revendication 6, comprenant l'enchaînement linéaire séquence comprenant au moins un résidu lysine-séquence 106-116 de la β-hCG.

**9.** Structure peptidique selon la revendication 8, comprenant l'enchâinement linéaire séquence 46-55 de l'α-hCGH - séquence 106-116 de la β-hCG.

**10.** Immunogène synthétique comprenant une structure peptidique selon l'une quelconque des revendications précédentes, couplée à un porteur.

**11.** Immunogène synthétique selon la revendication 10, dans lequel le porteur est l'anatoxine tétanique.

**12.** Immunogène synthétique selon la revendication 10 ou la revendication 11, dans lequel le porteur est couplé avec un résidu lysine de la séquence comprenant au moins un résidu lysine.

**13.** Immunogène synthétique selon la revendication 12, dans lequel le couplage est réalisé par le glutaraldéhyde.

**14.** Immunogène synthétique selon la revendication 10 ou la revendication 11, dans lequel le porteur est couplé avec l'un des acides aminés de la séquence 106-116 de la β-hCG.

**15.** Immunogène synthétique selon la revendication 14, dans lequel le porteur est couplé avec l'un des résidu acide aspartique de la séquence 106-116 de la β-hCG.

**16.** Immunogène synthétique selon la revendication 10 comprenant une structure peptidique selon l'une quelconque des revendications 1 à 9, couplée à une matrice de polylysine

**17.** Procédé de préparation d'un vaccin antifertilité, caractérisé en ce que l'on met une structure peptidique selon l'une quelconque des revendications 1 à 9 ou un immunogène synthétique selon l'une quelconque des revendications 10 à 16, sous une forme pharmaceutiquement acceptable.

**18.** Procédé de préparation d'un vaccin anti-hCG, caractérisé en ce que l'on met une structure peptidique selon l'une quelconque des revendications 1 à 9 ou un immunogène synthétique comprenant une telle structure peptidique couplée à un porteur sous une forme pharmaceutiquement acceptable.

## Claims

**Claims for the following Contracting States : AT, BE, CH, DE, FR, GB, IT, LI, LU, NL, SE**

**1.** A peptide structure comprising the combination
- of the sequence 106-116 of β-hCG and
- of a sequence of at least 5 amino acids containing at least one lysine residue.

**2.** A peptide structure according to claim 1, comprising
- the sequence 106-116 of β-hCG
- a sequence of at least 5 amino acids of an α-CG containing at least one lysine residue.

**3.** A peptide structure according to claim 2, comprising
- the sequence 106-116 of β-hCG
- the sequence 46-55 of α-hCG.

**4.** A peptide structure according to claim 2, comprising
- the sequence 106-116 of β-hCG
- the sequence 45-49 of α-hCG.

**5.** A peptide structure according to claim 2, comprising
- the sequence 106-116 of β-hCG
- the sequence 43-55 of α-hCG.

**6.** A peptide structure according to claims 1 to 5, in which the two sequences are combined in the form of a linear arrangement.

**7.** A peptide structure according to claim 6, in which the lysine residue is separated by at least 4 amino acids from the sequence 106-116 of β-CG or β-LH.

**8.** A peptide structure according to claim 6, comprising the linear arrangement: sequence containing at least one lysine residue - sequence 106-116 of β-hCG.

**9.** A peptide structure according to claim 8, comprising the linear arrangement: sequence 46-55 of α-hCGH - sequence 106-116 of β-hCG.

**10.** A synthetic immunogen comprising a peptide structure according to any one of the preceding claims, coupled to a carrier.

**11.** A synthetic immunogen according to claim 10, in which the carrier is tetanus toxoid.

**12.** A synthetic immunogen according to claim 10 or claim 11, in which the carrier is coupled to a lysine residue of the sequence containing at least one lysine residue.

**13.** A synthetic immunogen according to claim 12, in which the coupling is carried out by glutaraldehyde.

**14.** A synthetic immunogen according to claim 10 or claim 11, in which the carrier is coupled with one of the amino acids of the sequence 106-116 of β-hCG.

**15.** A synthetic immunogen according to claim 14, in which the carrier is coupled with one of the aspartic acid residues of the sequence 106-116 of β-hCG.

**16.** A synthetic immunogen according to claim 10, comprising a peptide structure according to any one of claims 1 to 9, coupled to a polylysine matrix.

**17.** An antifertility vaccine comprising a peptide structure according to any one of claims 1 to 9 or a synthetic immunogen according to any one of claims 10 to 16, and a pharmaceutically acceptable vehicle.

**18.** An anti-hCG vaccine comprising a peptide structure according to any one of claims 1 to 9 or a synthetic immunogen comprising such a peptide structure coupled to a carrier and a pharmaceutically acceptable vehicle.

**19.** An anti-hCG vaccine according to claim 18 for use in a birth control process.

**Claims for the following Contracting State : ES**

**1.** A process for preparing a peptide structure comprising the combination of
- the sequence 106-116 of $\beta$-hCG and
- a sequence of at least 5 amino acids containing at least one lysine residue, characterised in that the structure is prepared by a peptide synthesis comprising the successive coupling of different amino acid residues.

**2.** A process according to claim 1, in which a peptide structure is prepared comprising
- the sequence 106-116 of $\beta$-hCG and
- a sequence of at least 5 amino acids of an $\alpha$-CG containing at least one lysine residue.

**3.** A process according to claim 2, in which a peptide structure is prepared comprising
- the sequence 106-116 of $\beta$-hCG
- the sequence 46-55 of $\alpha$-hCG.

**4.** A process according to claim 2, in which a peptide structure is prepared comprising
- the sequence 106-116 of $\beta$-hCG
- the sequence 45-49 of $\alpha$-hCG.

**5.** A process according to claim 2, in which a peptide structure is prepared comprising
- the sequence 106-116 of $\beta$-hCG
- the sequence 43-55 of $\alpha$-hCG.

**6.** A process according to claim 1, in which a peptide structure is prepared in which the two sequences are combined in the form of a linear arrangement.

**7.** A process according to claim 6, in which a peptide structure is prepared, in which the lysine residue is separated by at least 4 amino acids from the sequence 106-116 of $\beta$-CG or $\beta$-LH.

**8.** A process according to claim 6, in which a peptide structure is prepared comprising the linear arrangement: sequence containing at least one lysine residue - sequence 106-116 of $\beta$-hCG.

**9.** A process according to claim 8, in which a peptide structure is prepared comprising the linear arrangement: sequence 46-55 of $\alpha$-hCGH - sequence 106-116 of $\beta$-hCG.

**10.** A process for preparing a synthetic immunogen comprising a peptide structure obtained by a process according to any one of the preceding claims, characterised in that the peptide structure is coupled to a carrier.

**11.** A process according to claim 10, in which the carrier is tetanus toxoid.

**12.** A process according to claim 10, in which the carrier in coupled to a lysine residue of the sequence comprising at least one lysine residue.

**13.** A process according to claim 12, in which the coupling is carried out by means of glutaraldehyde.

**14.** A process according to claim 10 or claim 13, in which the carrier is coupled to one of the amino acids of the sequence 106-116 of β-hCG.

**15.** A process according to claim 14, in which the carrier is coupled to one of the aspartic acid residues of the sequence 106-116 of β-hCG.

**16.** A process for preparing an antifertility vaccine, characterised in that a peptide structure obtained by means of a process according to any one of claims 1 to 9 or a synthetic immunogen obtained by a process according to any one of claims 10 to 15 is put into a pharmaceutically acceptable form.

**17.** A process for preparing an anti-hCG vaccine, characterised in that a peptide structure obtained by a process according to any one of claims 1 to 9 or a synthetic immunogen, comprising such a peptide structure coupled to a carrier, is put into a pharmaceutically acceptable form.

**18.** A process for preparing a synthetic immunogen, characterised in that a peptide structure is synthesised by a process according to any one of claims 1 to 9, on a polylysine matrix.

**Claims for the following Contracting State : GR**

**1.** A peptide structure comprising the combination of the sequence 106-116 of β-hCG and of a sequence of 5 amino acids containing at least one lysine residue.

**2.** A peptide structure according to claim 1, comprising
- the sequence 106-116 of β-hCG
- a sequence of at least 5 amino acids of an α-CG containing at least one lysine residue.

**3.** The peptide structure according to claim 2, comprising
- the sequence 106-116 of β-hCG
- the sequence 46-55 of α-hCG.

**4.** The peptide structure according to claim 2, comprising
- the sequence 106-116 of β-hCG
- the sequence 45-49 of α-hCG.

**5.** The peptide structure according to claim 2, comprising
- the sequence 106-116 of β-hCG
- the sequence 43-55 of α-hCG.

**6.** A peptide structure according to claims 1 to 5, in which the two sequence are combined in the form of a linear arrangement.

**7.** A peptide structure according to claim 6, in which the lysine residue is separated by at least 4 amino acids from the sequence 106-116 of β-CG or β-LH.

**8.** A peptide structure according to claim 6, comprising the linear arrangement: sequence containing at least one lysine residue - sequence 106-116 of β-CG.

**9.** A peptide structure according to claim 8, comprising the linear arrangement: sequence 46-55 of α-hCGH - sequence 106-116 of β-hCG.

**10.** A synthetic immunogen comprising a peptide structure according to any one of the preceding claims, coupled to a carrier.

**11.** A synthetic immunogen according to claim 10 in which the carrier is tetanus toxoid.

**12.** A synthetic immunogen according to claim 10 or claim 11, in which the carrier is coupled with a lysine residue of the sequence containing at least one lysine residue.

**13.** A synthetic immunogen according to claim 12, in which the coupling is carried out by means of glutaraldehyde.

**14.** A synthetic immunogen according to claim 10 or claim 11, in which the carrier is coupled with one of the amino acids of the sequence 106-116 of β-hCG.

**15.** A synthetic immunogen according to claim 14, in which the carrier is coupled with one of the aspartic acid residues of the sequence 106-116 of β-hCG.

**16.** A synthetic immunogen according to claim 10, comprising a peptide structure according to any one of claims 1 to 9, coupled to a polylysine matrix.

**17.** A process for preparing an antifertility vaccine, characterised in that a peptide structure according to any one of claims 1 to 9 or a synthetic immunogen according to any one of claims 10 to 16 is put into pharmaceutically acceptable form.

**18.** A process for preparing an anti-hCG vaccine, characterised in that a peptide structure according to any one of claims 1 to 9 or a synthetic immunogen, comprising such a peptide structure coupled to a carrier, is put into a pharmaceutically acceptable form.

**Patentansprüche**
**Patentansprüche für folgende Vertragsstaaten : AT, BE, CH, DE, FR, GB, IT, LI, LU, NL, SE**

**1.** Peptidstruktur umfassend die Kombination aus
- der Sequenz 106-116 von β-hCG und
- einer Sequenz von mindestens 5 Aminosäuren, die mindestens einen Lysinrest umfaßt.

**2.** Peptidstruktur nach Anspruch 1, umfassend
- die Sequenz 106-116 von β-hCG
- eine Sequenz von mindestens 5 Aminosäuren eines α-CG, die mindestens einen Lysinrest umfaßt.

**3.** Peptidstruktur nach Anspruch 2, umfassend
- die Sequenz 106-116 von β-hCG
- die Sequenz 46-55 von α-hCG.

**4.** Peptidstruktur nach Anspruch 2, umfassend
- die Sequenz 106-116 von β-hCG
- die Sequenz 45-49 von α-hCG.

**5.** Peptidstruktur nach Anspruch 2, umfassend
- die Sequenz 106-116 von β-hCG
- die Sequenz 43-55 von α-hCG.

**6.** Peptidstruktur nach den Ansprüchen 1 bis 5, worin die beiden Sequenzen in Form einer linearen Kette vereinigt sind.

**7.** Peptidstruktur nach Anspruch 6, worin der Lysinrest durch mindestens 4 Aminosäuren von der Sequenz 106-116 von β-CG oder von β-LH getrennt ist.

**8.** Peptidstruktur nach Anspruch 6, umfassend die mindestens einen Lysinrest enthaltende lineare Kettensequenz - Sequenz 106-116 von β-hCG.

**9.** Peptidstruktur nach Anspruch 8, enthaltend die lineare Kettensequenz 46-55 von α-hCGH Sequenz 106-116 von β-hCG.

**10.** Synthetisches Immunogen umfassend eine Peptidstruktur nach einem der vorhergehenden Ansprüche, die an einen Träger gekuppelt ist.

**11.** Synthetisches Immunogen nach Anspruch 10, worin der Träger Tetanus-Anatoxin ist.

**12.** Synthetisches Immunogen nach Anspruch 10 oder Anspruch 11, worin der Träger mit einem Lysinrest der Sequenz gekuppelt ist, die mindestens einen Lysinrest umfaßt.

**13.** Synthetisches Immunogen nach Anspruch 12, worin die Kupplung durch Glutaraldehyd bewirkt worden ist.

**14.** Synthetisches Immunogen nach Anspruch 10 oder Anspruch 11, worin der Träger mit einer der Aminosäuren der Sequenz 106-116 von $\beta$-hCG gekuppelt ist.

**15.** Synthetisches Immunogen nach Anspruch 14, worin der Träger mit einem der Asparaginsäurereste der Sequenz 106-116 von $\beta$-hCG gekuppelt ist.

**16.** Synthetisches Immunogen nach Anspruch 10, umfassend eine Peptidstruktur nach einem der Ansprüche 1 bis 9, gekuppelt an eine Polylysin-Matrix.

**17.** Antifertilitäts-Impfstoff enthaltend eine Peptidstruktur nach einem der Ansprüche 1 bis 9 oder ein synthetisches Immunogen nach einem der Ansprüche 10 bis 16 und einen geeigneten pharmazeutischen Träger.

**18.** Anti-hCG-Impfstoff enthaltend eine Peptidstruktur nach einem der Ansprüche 1 bis 9 oder ein synthetisches Immunogen umfassend eine an einen Träger gekuppelte solche Peptidstruktur und einen pharmazeutisch annehmbaren Träger.

**19.** Anti-hCG-Impfstoffnach Anspruch 18 zur Verwendung bei einem Verfahren zur Geburtenkontrolle.

**Patentansprüche für folgenden Vertragsstaat : ES**

**1.** Verfahren zur Herstellung einer Peptidstruktur umfassend die Kombination aus
- der Sequenz 106-116 von $\beta$-hCG und
- einer Sequenz aus mindestens 5 Aminosäuren, die mindestens einen Lysinrest umfaßt, **dadurch gekennzeichnet**, daß man die Struktur durch Peptidsynthese herstellt umfassend die aufeinanderfolgende Kupplung der verschiedenen Aminosäurereste.

**2.** Verfahren nach Anspruch 1, worin man eine Peptidstruktur herstellt, welche
- die Sequenz 106-116 von $\beta$-hCG
- eine Sequenz aus mindestens 5 Aminosäuren eines $\alpha$-CG, die mindestens einen Lysinrest aufweist, umfaßt.

**3.** Verfahren nach Anspruch 2, worin man eine Peptidstruktur herstellt, welche
- die Sequenz 106-116 von $\beta$-hCG
- die Sequenz 46-55 von $\alpha$-hCG umfaßt.

**4.** Verfahren nach Anspruch 2, worin man eine Peptidstruktur herstellt, welche
- die Sequenz 106-116 von $\beta$-hCG
- die Sequenz 45-49 von $\alpha$-hCG umfaßt.

**5.** Verfahren nach Anspruch 2, worin man eine Peptidstruktur herstellt, welche
- die Sequenz 106-116 von $\beta$-hCG
- die Sequenz 43-55 von $\alpha$-hCG umfaßt.

**6.** Verfahren nach Anspruch 1, worin man eine Peptidstruktur herstellt, in der die beiden Sequenzen in Form einer linearen Kette verbunden sind.

**7.** Verfahren nach Anspruch 6, worin man eine Peptidstruktur herstellt, bei der der Lysinrest durch mindestens 4 Aminosäuren von der Sequenz 106-116 von $\beta$-CG oder $\beta$-LH getrennt ist.

**8.** Verfahren nach Anspruch 6, worin man eine Peptidstruktur herstellt, welche die lineare Kette (Sequenz, die mindestens einen Lysinrest umfaßt)-(Sequenz 106-116 von β-hCH) umfaßt.

**9.** Verfahren nach Anspruch 8, worin man eine Peptidstruktur herstellt, welche die lineare Kette (Sequenz 46-55 von α-hCGH) - (Sequenz 106-116 von β-hCH) umfaßt.

**10.** Verfahren zur Herstellung eines synthetischen Immunogens umfassend eine Peptidstruktur erhalten nach einem Verfahren gemäß einem der vorhergehenden Ansprüche, **dadurch gekennzeichnet**, daß man die Peptidstruktur mit einem Träger kuppelt.

**11.** Verfahren nach Anspruch 10, worin der Träger Tetanus-Anatoxin ist.

**12.** Verfahren nach Anspruch 10, worin der Träger mit einem Lysinrest der mindestens einen Lysinrest umfassenden Sequenz gekuppelt wird.

**13.** Verfahren nach Anspruch 12, worin die Kupplung durch Glutaraldehyd bewirkt wird.

**14.** Verfahren nach Anspruch 10 oder Anspruch 13, worin der Träger mit einer der Aminosäuren der Sequenz 106-116 von β-hCG gekuppelt wird.

**15.** Verfahren nach Anspruch 14, worin der Träger mit einem der Asparaginsäurereste der Sequenz 106-116 von β-hCG gekuppelt wird.

**16.** Verfahren zur Herstellung eines Antifertilitäts-Impfstoffs, **dadurch gekennzeichnet**, daß man eine nach einem Verfahren gemäß einem der Ansprüche 1 bis 9 erhaltene Peptidstruktur oder ein nach einem Verfahren gemäß einem der Ansprüche 10 bis 15 erhaltenes synthetisches Immunogen in eine pharmazeutisch annehmbare Form bringt.

**17.** Verfahren zur Herstellung eines Anti-hCG-Impfstoffs, **dadurch gekennzeichnet,** daß man eine nach einem Verfahren gemäß einem der Ansprüche 1 bis 9 erhaltene Peptidstruktur oder ein synthetisches Immunogen, welches eine solche an einen Träger gekuppelte Peptidstruktur umfaßt, in eine pharmazeutisch annehmbare Form bringt.

**18.** Verfahren zur Herstellung eines synthetischen Immunogens, **dadurch gekennzeichnet,** daß man eine Peptidstruktur nach einem Verfahren gemäß einem der Ansprüche 1 bis 9 auf einer Polylysin-Matrix synthetisiert.

**Patentansprüche für folgenden Vertragsstaat : GR**

**1.** Peptidstruktur umfassend die Kombination aus
- der Sequenz 10-116 von β-hCG und
- einer Sequenz von mindestens 5 Aminosäuren, die mindestens einen Lysinrest umfaßt.

**2.** Peptidstruktur nach Anspruch 1, umfassend
- die Sequenz 106-116 von β-hCG
- eine Sequenz von mindestens 5 Aminosäuren eines α-CG, die mindestens einen Lysinrest umfaßt.

**3.** Peptidstruktur nach Anspruch 2, umfassend
- die Sequenz 106-116 von β-hCG
- die Sequenz 46-55 von α-hCG.

**4.** Peptidstruktur nach Anspruch 2, umfassend
- die Sequenz 106-116 von β-hCG
- die Sequenz 45-49 von α-hCG.

**5.** Peptidstruktur nach Anspruch 2, umfassend
- die Sequenz 106-116 von β-hCG

- die Sequenz 43-55 von α-hCG.

6. Peptidstruktur nach den Ansprüchen 1 bis 5, worin die beiden Sequenzen in Form einer linearen Kette vereinigt sind.

7. Peptidstruktur nach Anspruch 6, worin der Lysinrest durch mindestens 4 Aminosäuren von der Sequenz 106-116 von β-CG oder von β-LH getrennt ist.

8. Peptidstruktur nach Anspruch 6, umfassend die mindestens einen Lysinrest enthaltende lineare Kettensequenz - Sequenz 106-116 von β-hCG.

9. Peptidstruktur nach Anspruch 8, enthaltend die lineare Kettensequenz 46-55 von α-hCGH - Sequenz 106-116 von β-hCG.

10. Synthetisches Immunogen umfassend eine Peptidstruktur nach einem der vorhergehenden Ansprüche, die an einen Träger gekuppelt ist.

11. Synthetisches Immunogen nach Anspruch 10, worin der Träger Tetanus-Anatoxin ist.

12. Synthetisches Immunogen nach Anspruch 10 oder Anspruch 11, worin der Träger mit einem Lysinrest der Sequenz gekuppelt ist, die mindestens einen Lysinrest umfaßt.

13. Synthetisches Immunogen nach Anspruch 12, worin die Kupplung durch Glutaraldehyd bewirkt worden ist.

14. Synthetisches Immunogen nach Anspruch 10 oder Anspruch 11, worin der Träger mit einer der Aminosäuren der Sequenz 106-116 von β-hCG gekuppelt ist.

15. Synthetisches Immunogen nach Anspruch 14, worin der Träger mit einem der Asparaginsäurereste der Sequenz 106-116 von β-hCG gekuppelt ist.

16. Synthetisches Immunogen nach Anspruch 10, umfassend eine Peptidstruktur nach einem der Ansprüche 1 bis 9, gekuppelt an eine Polylysinmatrix.

17. Verfahren zur Herstellung eines Antifertilitäts-Impfstoffs, **dadurch gekennzeichnet**, daß man eine Peptidstruktur nach einem der Ansprüche 1 bis 9 oder ein synthetisches Immunogen nach einem der Ansprüche 10 bis 16 in eine pharmazeutisch annehmbare Form bringt.

18. Verfahren zur Herstellung eines Anti-hCG-Impfstoffs, **dadurch gekennzeichnet,** daß man eine Peptidstruktur nach einem der Ansprüche 1 bis 9 oder ein synthetisches Immunogen, welches eine solche an einen Träger gekuppelte Peptidstruktur umfaßt, in eine pharmazeutisch annehmbare Form bringt.